# EUROPEAN PATENT APPLICATION

(11) **EP 2 116 254 A1**
(43) Date of publication of application: **11.11.2009**
(21) Application number: 07852028.5
(22) Date of filing: 16.10.2007
(51) Int. Cl.: A61K 38/00, A61K 39/00, C12N 5/08, A61P 35/00

(54) **TUMOR VACCINE, A METHOD FOR PRODUCING A TUMOR VACCINE AND A METHOD FOR CARRYING OUT ANTITUMOR IMMUNOTHERAPY**

(30) Priority: 07.03.2007 EA 200700598
(71) Applicant: Lokhov, Petr Genrievich, Moscow 113452 (RU)
(72) Inventor: Lokhov, Petr Genrievich, Moscow 113452 (RU)
(74) Representative: Hatzmann, Martin
(86) International application number: PCT/RU2007/000572
(87) International publication number: WO 2008/108679

(57) **Abstract**

The group of inventions relates to medical engineering, in particular to the immunotherapy of cancer patients. The inventive tumor vaccine based on surface tumor antigens comprises a mixture of surface tumor antigens. The method for producing a tumor vaccine consists in cultivating tumor cells and in separating surface tumor antigens. The primary culture of living tumor cells, which is pre-washed of a growth medium, is exposed to a proteases action vital for cells and the thus released surface tumor antigens are separated. The primary culture of living tumor cells is repeatedly treated by proteases at intervals necessary for recovering the surface tumor antigens by means of cells. The surface tumor antigens are accumulated until a dose thereof, required for vaccination, is obtained, the composition of the thus obtained surface tumor antigens is tested. Trypsin can be used as protease. The method for carrying out antitumor immunotherapy consists in administrating the tumor vaccine in a patient body. The inventive group of inventions makes it possible to increase the effectiveness of treatment of oncological diseases by enhancing an antitumor immune response.

## Description

### FIELD OF THE INVENTION

This group of inventions belongs to the medical technologies namely to the immunotherapy of oncological patients and it could be used in medicine for the therapy of oncological diseases and for the prevention of relapses.

### BACKGROUND

The restricted possibilities of treatment of oncological diseases by means of methods of surgery, chemotherapy and radiotherapy make most demanded the search of new methods of cure of oncological patients. In particular there is a belief that the development of methods of immunotherapy gives good prospects, the principle of immunotherapy being the enhancement of anti-tumor defense innate in the immunity of humans.

One of the effective methods of immunotherapy is thought to be the vaccination, the effectiveness of which depends from the strength of immune response caused by tumor antigens either present in the vaccine composition, or necessary during the various stages of vaccine production (for example, vaccines prepared on the base of anti-idiotypic antibodies, dendritic cells etc). So, the isolation of tumor antigens is the necessary precondition for the development of anti-tumor vaccines.

There are well-known different methods of preparation of anti-tumor vaccines.

Some vaccines are prepared using particular antigens of the tumor cell (TC) - that is peptides, heat shock proteins, polysaccharides and other substances. In particular is well-known the method of fabrication of tumor peptide antigens by means of synthesis. Thus the vaccine consisting of 9 amino acid long synthetic peptide demonstrated good results during clinical trials on patients with myeloid leukemia (Williams R., "Harnessing the Immune System: The Promise and Potential of Cancer Vaccines", Oncolog., 2005, v. 50, Nº 4). Other peptides demonstrated non-uniform results. For example, the peptide from the *gp100* protein, which demonstrated good preliminary results, did not cause immune response sufficient to resist tumor development in the patients (Yu Z., Restifo N.P., "Cancer Vaccines: Progress Reveals New Complexities", J. Clin. Invest., 2002, v. 110, 289-294).

The method using tumor carbohydrates as antigens provoking the immune response is well-known. But the effectiveness of similar antigens was demonstrated only in case of single tumor cells and early stage metastases (Franco A., "CTL-Based cancer Preventive/Therapeutic Vaccines for Carcinomas: Role of Tumour-Associated Carbohydrate Antigens", Scand. J. Immunol., 2005, v. 61, 391-397).

It's necessary to note that vaccines based on definite antigens possess the essential shortcoming. TC are characterized not only by the plurality of antigens, their high mutation rate and constantly acting mechanism of cell selection in the organism leads to the appearance of new and to the modification of already existing antigens of TC. Thus becomes evident the advantage of the use of auto-vaccines (vaccines based on the patient's own TC), which contain the whole spectrum of antigens inducing the immune response, including individual and disease stage-specific antigens, characterizing the development of tumor process of the concrete patient.

The method of preparation of tumor antigens using exosomes is well-known. Exosomes are few nanometers in diameter membrane vesicles, secreted by many types of cells, including tumor cells (see e.g. the paper Wolfers J. et al., "Tumor-derived exosomes are a source of shared tumor rejection antigens for CTL cross-priming", Nat. Med., 2001, v. 7, 297-303), T-and B-lymphocytes (Raposo G. et al., "B lymphocytes secrete antigenpresenting vesicles", J. Exp. Med., 1996, v. 183, 1161-1172) and dendritic cells (Zitvogel L. et al., "Eradication of established murine tumors using a novel cell-free vaccine: dendritic cell-derived exosomes", Nature Med., 1998, v. 4, 594-600).

The shortcoming of this method is the low level of enrichment of exosomes with surface proteins which are potential antigens for performing immunotherapy. It is well known that exosomes contain mainly the cytosol proteins and proteins of endosomal compartments (see e.g. the paper Thery C. et al., "Exosomes: composition, biogenesis and function" Reviews Immunology, 2002, v. 2, 569-579). The second well-known shortcoming of the method is a long time necessary for the accumulation of exosomes, which makes necessary the presence of TC in the growth medium and makes necessary the purification of antigens from the growth medium components.

It is well-known the method of preparation of anti-tumor vaccine, which is based on the introduction into the organism of the DNA encoding the antigens instead of antigens themselves. Antigen-presenting cells absorb the DNA, produce the tumor antigen and present it on the cell surface bound with the main hystocompatibility complex, which is able to activate the cytotoxic T-lymphocytes. Thus good results were obtained on animal models, using vaccines based on virus vectors (see e.g. the paper Yu Z., Restifo N.P., "Cancer Vaccines: Progress Reveals New Complexities", J. Clin. Invest, 2002, v. 110, 289-294).

The shortcoming of this method is the reaction of the immune system induced by the virus vectors themselves, which results in the absence of evident positive effects of anti-tumor vaccination in humans.

It is well known the method of preparation of anti-tumor vaccines which presupposes the use of whole tumor cells as antigens, obtained as well from the patient's own tumor (autologous vaccines) as also from the tumor of other patients (allogeneic vaccines). Cells are preliminarily inactivated with ionizing radiation. The advantage of such vaccines is the absence of necessity of identification and isolation of concrete antigens. So the cells mixed with BCG-vaccine as adjuvant were used against colorectal cancer, melanoma and kidney carcinoma (see Armstrong A.C., Eaton D., Ewing J.C., "Cellular Immunotherapy for Cancer", Brit. Med. J., 2001, v. 3323, 1289-1293).

The shortcomings of this method are the necessity of inactivation of tumor cells and the low suitability of antigens located on the whole tumor cell surface for the phagocytosis and processing by antigen-presenting cells.

From the US patent 6039941 (index C12N 15/09, A61K 31/00, published on 21.03.2000) is well known the method of preparation of highly immunogenic anti-tumor vaccine from TC obtained through introduction by means of genetic engineering of the genes encoding surface proteins possessing immunostimulating activity.

The shortcomings of this method are, first, - the necessity of inactivation of tumor cells, second, - the fact that antigens on the tumor cell surface are hardly suitable for the phagocytosis and processing by antigen-presenting cells.

From the International Application WO 02053176 (index A61K 39/00; C12N 5/06; A61K 39/00; C12N 5/06, published on 11.07.2002) it is well known the method of preparation of the anti-tumor vaccine based on the lysates of cultivated TC. The advantage of these vaccines is that they do not need inactivation by ionizing radiation and that disaggregated cell antigens are more suitable, unlike intact TC, for the phagocytosis and processing by antigen-presenting cells, which could result in more pronounced immune response.

The shortcoming of this method is the fact that the main part of the TC lysate consists of intracellular proteins and that caused by these proteins immune response does not possess anti-tumor activity, because intracellular proteins of tumor cells are inaccessible for the immune system.

The nearest analogue of this method (application for the patent) is the method of preparing of the anti-tumor vaccine described in the US patent 5993829 (index A61P 35/00, C07K 14/47, published on 30.11.1999). Analogous methods including the tumor cells cultivation and isolation of their surface antigens are also described in US 6338853 (index A61P 35/00, C07K 14/47, published on 15.01.2002), US 5030621 (published on 09.07.1991) and US 5635188 (published on 03.06.1997) patents.

The well-known method presupposes the cultivation of tumor cells in serum-free growth medium and the isolation out of growth medium of the surface cell antigens, which tumor cells lose during the cultivation process. After the purification the collected antigens are used as antigens of the anti-tumor vaccine. The advantage of such vaccines is the high content of tumor antigens from cell surface, which are accessible (are not hidden inside the cell) for the action of the immune system. The shortcomings of this method are:
- the low output of antigens because of spontaneous, not depending on external influence, loss of antigens by cultivated TC;
- the obtaining of material with low content of desired product (antigens) and with high amount of impurities, due to the fact that the process of antigens accumulation, presupposes the incubation of TC in growth medium during many hours (e.g. three-hours incubation in RPMI-1640 medium according the US patent 6338853), which assumes the accumulate of antigens in the growth medium containing more than 40 substances (amino acids, salts, buffer agents, vitamins, glucose etc), containing also products of metabolism and other substances secreted by TC during the cultivation process;
- the analysis of composition of antigen mixture necessitates the preliminary purification of the preparation from the components of growth medium and from products of cell metabolism;
- the isolation of antigen dose necessary for the vaccination assumes the proliferation of TC through prolonged cultivation, which leads to the distortion of the composition of antigen mixture and makes vaccination less effective;
- the amount of antigens necessary for the vaccination could be obtained accordingly to this method only through the process of cultivation of tumor cell, that is, the extraction of antigens from the isolated (with no cultivation) cells accordingly to this method is impossible;
- the vaccine is expensive which is due to the long cultivation of TC and the necessity to purify surface tumor antigens.

There are also well known anti-tumor vaccines which basic composition is a mixture of different antigens or a mixture of antigens and different immunostimulating substances.

Out of International Application WO 2004012685 (index A61K 39/00, published on 12.02.2004) is known the anti-tumor vaccine on the base of surface tumor antigens which are "lost" by the cells during the cultivation process. The preparation of this vaccine assumes the possibility of acceleration of the process of secretion of antigens by the cells into the growth medium after different influences which can somewhat rise the effectiveness of the vaccine. But this vaccine also has shortcomings inherent to other vaccines obtained through the cultivation of TC in serum-free media.

The most near analogue of the Patent Application, which is described here, - is the vaccine on the basis of surface tumor antigens, disclosed in the US Patent 5993829 (index A61P 35/00, C07K 14/47, published on 30.11.1999). Analogous vaccines are also described in the US Patents 6338853 (index A61P 35/00, C07K 14/47, published on 15.01.2002), US 5030621 (published on 09.07.1991), US 5635188 (published on 3.06.1997). The shortcoming of this well-known vaccine is it's high cost which depends on the duration of the TC cultivation and on the presence of purification stage, as it was noted earlier.

There are also well-known different methods of performing of the anti-tumor therapy.

There are known methods of performing anti-tumor immunotherapy with monovalent vaccines, including the introduction into the organism of definite tumor antigens. To such vaccines belong, for example, vaccines on the basis of synthetic peptides, heat shock proteins, polysaccharides and other substances. These methods are disclosed in particular in patents WO 2005083074 (index A61K 31/7088; A61P 35/00; C07K 7/06, published on 09.09.2005) and RU 2271831 (A61K 39/385; A61K 39/39; A61K 51/00, published on 20.03.2006).

The shortcoming of these methods is the weak specificity of induced anti-tumor immunity because of the fact that certain antigens are not specific for the tumor of concrete patient, because they were discovered as statistically common antigens for tumor types.

There is well known the method of performing of the anti-tumor therapy including the injection into the organism of the whole inactivated tumor cells and also of whole tumor cells with reinforced immunogenicity (see, for example, US Patent 6039941, index C12N 15/09, A61K 31/00, published on 21.03.2000).

The shortcoming of this method of immunotherapy is the low specificity of induced anti-tumor response because antigens on the surface of tumor cells are little suitable for the phagocytosis and following processing by antigen-presenting cells. This method also presupposes the *in vitro* proliferation of the cells necessary to obtain the dose of antigen adequate for the vaccination, which also leads to the changes of antigen composition and to the decrease of the effectiveness of the immunotherapy.

Out of the International Application WO 02053176 (index A61K 39/00; C12N 5/06; A61K 39/00; C12N 5/06, published on 11.07.2002) is well known the method of performing of anti-tumor immunotherapy including the introduction into the organism of the tumor cell lysates.

The shortcoming of this method of vaccination is the low specificity of induced anti-tumor immunity. In this case the vaccine besides the surface tumor antigens responsible for the immune response contains also intracellular proteins constituting the main part of the lysate. So the immunization against the mass of ballast proteins occurs and the "blurring" of tumor-specificity of the immune response takes place. This method also presupposes the *in vitro* proliferation of the cells necessary to obtain the adequate for the vaccination antigen dose, which leads to the changes of antigen composition of the tumor cells and consequently to the decrease of the effectiveness of anti-tumor therapy.

There are known methods based on the combination of particular tumor antigens and also on combinations of different antigens and immunostimulants of various kinds.

The most near analogue of the represented here method of performing the anti-tumor therapy is the method disclosed in US Patent 5993829 (index A61P 35/00, C07K 14/47, published on 30.11.1999). Analogous methods are described in patents US 6338853 (index A61P 35/00, C07K 14/47, published on 15.01.2002), US 5030621 (published on 09.07.1991), US 5635188 (published on 03.06.1997) and WO 2004012685 (index A61K 39/00, published on 12.02.2004). The method includes the injection into the patient's organism of the vaccine obtained from the mixture of antigens, which are the fragments of surface proteins of tumor cells that were spontaneously lost by the cells during the cultivation in serum-free medium.

One of the shortcomings of this method is the low specificity of the induced immune response. This method presupposes the use of long cultivated TC, which leads to the changes of their antigen composition and to the decrease of the effectiveness of the vaccine. Another shortcoming is a relatively high cost of the method, due to the duration of cultivation time of the cells and to the necessity to purify isolated antigens from the components of growth medium.

### SUMMARY OF THE INVENTION

The technical result obtained through the use of this group of to be patented inventions consists in the enhancement of the effectiveness of the treatment of oncological diseases due to the enforcement of the anti-tumor immune response.

This technical result is secured with the use of anti-tumor vaccine on the basis of surface tumor antigens. Accordingly to the present invention the vaccine contains a mixture of surface tumor antigens, which are accumulated peptides from surface proteins of the live tumor cells, obtained through periodical, non-deadly action of the protease on the primary culture of live tumor cells.

In the preferential variant of realization of the invention the anti-tumor vaccine could be obtained through the use of trypsin chosen as the protease.

The before mentioned technical result is also obtained with the realization of the method of preparation of the anti-tumor vaccine including the cultivation of tumor cells and isolation of the surface tumor antigens. According to the present invention the primary culture of live tumor cells, preliminarily rinsed from the growth medium, is subjected to the non-deadly for cells action of the protease, the released surface tumor antigens are collected, in addition the treatment of the primary culture of live tumor cells with the protease is repeated after time intervals necessary for the recovery of surface tumor antigens by the cells, the surface tumor antigens are accumulated until the dose sufficient for the vaccination is reached, the composition of obtained surface tumor antigens is controlled.

In the preferential variant of realization of the invention the trypsin is used as the protease.

The before mentioned technical result is also obtained through the realization of the method of performing the anti-tumor immunotherapy, including the injection into the patient's organism of the vaccine which is obtained from the mixture of surface tumor antigens, these antigens being peptides obtained from the surface proteins of the tumor cells. Accordingly to the present invention the mixture of surface tumor antigens is used, which antigens are accumulated peptides from the surface proteins of the live tumor cells, antigens obtained through periodical, non-deadly for cells action of the protease on the primary culture of live tumor cells.

In the preferential variant of realization trypsin is used as the protease.

In order to obtain the more specific immune response surface tumor antigens of the patient's own tumor cells are used.

In other modes of realization of the invention surface tumor antigens of the cells of another patient are used.

In the preferential variant of realization of the method adjuvants are used as a part of vaccine composition.

### BRIEF DESCRIPTION OF THE DRAWINGS

Further the group of inventions is illustrated with concrete examples of realization and with accompanying figures representing the following.

Fig.1. - The scheme of realization of the method of preparation of the anti-tumor vaccine.

Fig.2. - The mass spectrum of surface antigens, obtained accordingly the second variant of realization of the invention, out of the primary tumor cells culture (A); the mass spectrum of surface antigens obtained out of the same cell culture after full reparation of the surface antigens and the repeated treatment of the cells with the protease (B); the mass spectrum of surface antigens obtained out of the same cell culture after partial reparation following the treatment of the cells with the protease (C).

Fig.3. - The mass spectra of the fragmentation of the surface antigens obtained accordingly the present invention.

Fig. 3A, 3B - spectra of the CID fragmentation of carbohydrate part of the antigens.

Fig. 3C, 3D - spectra of the CID fragmentation of peptide sequence of antigens with the identification of b/y ions.

Fig. 4. - The curve of the survival of mice bearing the inoculated tumor cell line H22 which have been vaccinated with the peptide mixture.

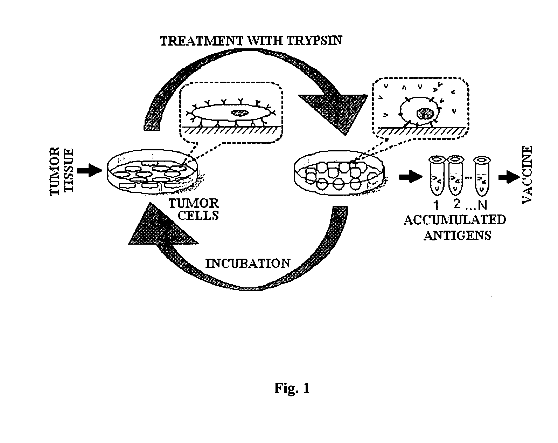

On the fig.1 a general scheme of preparation of the anti-tumor vaccine is drawn.

### PREFERRED EMBODIMENTS OF THE METHOD

Further the first example of realization of the method of preparation of the anti-tumor vaccine against the human hepatoma H22 inoculated to mice is given. The same culture of H22 cells is used to prepare the vaccine.
1. The growth medium is removed from the flask with the H22 cells culture, the cell monolayer is washed, using sterile physiological solution in volumes no less then half the volume of the growth medium. The washing should be performed no less then three times in order to remove completely the remainders of the growth medium.
   The next and also a crucial stage is the treatment of the cells with non-deadly protease concentration, usually with the trypsin (activity ∼3000 U/mg).
2. The 0.0001% trypsin solution is added to the cells monolayer, 1 ml of the solution is used for every 25 cm² of the flask surface.
3. The flask is incubated at 37°C. Between the 5-th and 7-th minutes of incubation the trypsin solution containing the split antigens from the cell surface is collected.
4. The fresh growth medium containing serum (10% usually) is added to the cells and the cultivation continues.
5. In order to obtain the necessary quantity of antigens p.p. 2-4 are repeated with 24 hours intervals.
6. For the assessment of usefulness of the antigens the mass spectrometry analysis is performed.
7. The accumulated antigens are used for the preparation of the vaccine.

The mass spectrometry analysis is done as follows. 140 µl of the antigens solution are mixed with 140 µl of ethanol and 720 µl of butanol, 15 µl of the Sepharose CL4B are added. The mixture is incubated during 45 min under slow stirring. After the incubation the sepharose is washed twice with the same ethanol-butanol solution and incubated for 30 min in 50% ethanol solution. The ethanol solution is collected and dried in the rotor-evaporating device. The obtained dry material is dissolved in 10 µl water and analyzed using MALDI-TOF mass spectrometry. 2 µl of the solution which undergoes analysis are mixed on the mass spectrometer target in 1:1 ratio with the saturated solution of 2,5 - dihydroxybenzoic acid containing 50% of acetonitrile and 0.5% of trifluoroacetic acid. The drops prepared on the target are air dried and the mass spectrometry analysis of peptides in the mass range 600-4000 Da is performed.

It is well known that the majority of tumor antigens, which are of some interest for the vaccine development, are found on the cell surface (see e.g. the review Bocchia M. et al., "Antitumor vaccination: where we stand", Hematologica, 2000, v. 85, 1172-1206). The non-deadly treatment of the primary cell culture with trypsin leads to the splitting of protein fragments from the cell surface (see Lokhov P.G., Archakov A.I., "Proteomic footprinting: a method for cells profiling by direct mass spectrometry", HUPO theses, 5-th Annual World Congress, 2006, abstract No 1201). So accordingly to this invention the action of trypsin as well of other proteases results in releasing of the fragments of tumor surface proteins into the solution. The released under the action of trypsin surface tumor antigens (fragments of the surface proteins) are mainly those suitable for the immunotherapy antigens which are used for the vaccination of oncological patients.

It is necessary to note that the harvesting of the trypsin solution, containing the peptides split from the cells, should be performed advisably before the detachment of the cells from the flask bottom, which permits to avoid the transfer of the cells into the sample and exclude additional purification steps.

The conditions of the treatment of the cells with a protease are defined experimentally for each type of tumor cells and the for every degree of activity of the used protease, they could significantly vary, that is from 0.0001% to 0.05% as for the protease concentration and from 30 seconds to 10 minutes as for the treatment time. In the case of the use of trypsin with a different degree of activity its concentration is changed in a right proportion to the increase or decrease of enzyme activity.

The cells after the treatment with non-deadly protease concentrations do not perish that gives the possibility to repeat the process of treatment of the primary cell culture with trypsin. In order to obtain the dose of surface tumor antigens necessary for the vaccination the process of treatment of the live cell culture with trypsin is repeated many times with intervals reaching up to 24 hours. During the intervals between the trypsin treatments the cells are incubated according to the protocol of incubation for the given cells (that is at 37°C in the CO₂ incubator, in the growth medium containing serum and necessary additional supplements).

The accumulated surface tumor antigens are treated accordingly with the technology of preparation of the given vaccine, namely, they could be subjected to the purification, concentration, analysis of their composition, and also they could be modified or mixed with adjuvants for the increase of immunogeneity.

In other variants of realization of the invention the protease treatment could be combined with the process of passage of the cells.

The trypsin solution could be prepared, using sterile physiological solution or any other appropriate saline solution.

Instead of trypsin other proteases could be used, for example, chemotrypsin etc.

In the case when cell suspension is used, it is necessary to harvest cells, before the accumulation of antigens, by means of centrifugation or use any other appropriate method.

The said analysis of composition of antigens, obtained accordingly to the first variant of realization, is performed using the protocol of mass spectrometry for glycosylated peptides (M. Tajiri et al., "Differential analysis of site-specific glycans on plasma and cellular fibronectins: application of a hydrophilic affinity method for glycopeptide enrichment", Glycobiology, 2005, v. 15, 1332-1340). It is due from one part to the fact that the protein fragments are usually glycosylated, from the other part namely the glycosylated peptides are the most immunogenic ones and represent obvious interest as vaccine antigens (see, for example, Franco A., "CTL-Based cancer Preventive/Therapeutic Vaccines for Carcinomas: Role of Tumour-Associated Carbohydrate Antigens", Scand. J. Immunol., 2005, v. 61, 391-397).

The desalting and the concentrating of glycosylated antigens could be done by any appropriate mean, in particular by liquid chromatography (HPLC).

In other variants of realization tumor cells could be isolated from biological liquids such as blood, urine, liquor, lymph, ascitic and pleural fluids.

This method of preparation of the anti-tumor vaccine could be applied to every type of cell culture, namely to the adherent culture, suspension culture, cell cultures with matrix and other substrata, to all variants of cell cocultivation, to organotype cultures and cell aggregates (granules, spheroids) and also to freshly isolated tumor cells and tumor tissue fragments.

Further the invention is illustrated by the second example of preparation of the anti-tumor vaccine and the method of it's preparation using the adherent primary culture of human colon cancer cells.
1. A fragment of colon tumor tissue obtained in result of surgical ablation of the tumor is transferred into the sterile test-tube with the RPMI 1640 medium containing antibiotics and then transported into the laboratory.
2. The tumor tissue is transferred under sterile conditions in the Petri Dish, sites of necrosis, blood clots and the remnants of fat and conjunctive tissues are mechanically removed.
3. The tissue is accurately cut into small fragments with scissors.
4. The fragments of tumor tissue are dissociated into small cell aggregates by vigorous pipetting, the fragments during this process are suspended in 10 ml of phosphate buffer silane (PBS).
5. The remaining big fragments of the tissue are let to fall on the test-tube bottom.
6. The cell aggregates still floating in the liquid are cautiously collected with a pipette and transferred into a new test-tube.
7. The test-tube is centrifuged for 5 minutes at 400 g, the supernatant is discarded. The pellet containing the cell aggregates is resuspended in the RPMI 1640 medium with the following supplements: insulin (20 µg/ml), transferrin (10 µg/ml), hydrocortisone (50 nM), epidermal growth factor (1 ng/ml), ethanolamine (10 µM), phosphoethanolamine (10 µM), triiodthyronine (100 pM), bovine serum albumin (2 mg/ml), glutamine (2 mM), sodium piruvate (0.5 mM), fetal bovine serum (5%) and then transferred into the 25 cm² flask for the cell cultivation.
8. The cultivation is performed at 37°C and 5% CO₂.
9. In order to isolate the tumor cells from the stromal cells the cultural flask is pressed against the vibro-stirring device during few seconds. Because of feeble adhesive qualities, tumor cells are detached and pass into the growth medium.
10. The medium with the floating cells is collected with a pipette and then is transferred into a new culture flask for the following cultivation at 37°C and 5% CO₂.
11. When tumor cell have reached 80% confluency the growth medium is removed from the flask and the cells are three times rinsed with 0.9% NaCl or with phosphate buffer silane, the volume of the rinsing solution should be no less then half the volume of the medium growth. As the result of rinsing the traces of the serum contained in the growth medium should be removed.
12. The 0.0001% trypsin solution (the activity ∼3000 U/mg) is added to the cells using 1 ml of the solution for every 25 cm² of the culture flask surface.
13. The flask is incubated at 37°C. Between the 5-th and the 7-th minutes of incubation the solution containing the surface antigens split from the cells is collected. Tumor cells during the collecting of the solution should remain attached to the bottom of the culture flask. If under the action of the trypsin some part of the cells is detached and began to float freely, the centrifugation at 400 g during 5 minutes is applied and the cell-free supernatant is used.
14. For the inactivation of the rests of trypsin in the culture flask it's necessary to add into the flask the freshly prepared growth medium containing fetal bovine serum and to continue the cultivation of the cells at 37°C and 5% CO₂.
15. The solution obtained according to the p.13 undergoes concentration in the vacuum concentrating device at 45°C. The solution could be preliminarily desalted by any appropriate mean, for example, by reverse phase chromatography, gel-filtration etc. For the removal of the rests of trypsin the solution could be preliminarily filtered through the filter which permits to pass the peptides with the molecular weight less then 4 kDa.
16. Steps in the p.p. 11-15 are repeated with 24 hours interval for the accumulation of the necessary quantity of antigens. The usefulness of these antigens for the vaccination is assessed with mass spectrometry.

The number of the treatments of the cells with the protease and the duration of intervals between treatments is controlled by analysis of the composition of the accumulated antigen mixture. If the antigenic composition begins to change and the increase of the time interval between the protease treatments of the cells does not allow to obtain the original antigenic composition corresponding to the freshly isolated tumor cells then the cells are not supposed to be suitable for the vaccine production.

The control of the composition of the accumulated antigen mixture is performed with mass spectrometry as it was indicated in the first example of realization of the method.

Any suitable method is used for the isolation of cells from tumor tissue. In case of the protease use for the dissociation of the tumor tissue aiming at the release of tumor cells there is a necessity to perform mass spectrometry analysis of the surface structures no earlier than 24 hours after the initiation of the primary tumor culture.

The surface tumor antigens obtained according to this invention should be specific for the tumor of the cell donor. But the cultivation of the cells distorts significantly the phenotype of the primary culture because of the impossibility of artificial re-creation *in vitro* of the conditions under which the tumor cells grew in the organism of the donor. So the composition of the accumulated antigens is subject to the obligatory control. Further is the example of assessment of the quality of antigens obtained accordingly the second variant of invention presented.

According to the data obtained after the recurring mass spectrometry analysis of the antigen mixture no less than 95% of masses of glycosylated peptides are reproduced, which one can consider as the criterion of identity of the two compared antigen mixtures. It is proposed to assess the antigens accumulated for the vaccination as suitable for this purpose by the presence of no less than 90% of masses of the glycosylated peptides common to freshly isolated tumor cells.

From the obtained experimental data it is known that the cultivation of the cells of the rectum tumor allows to prepare antigens suitable for the vaccination only using the first and second cell passages under the condition that passages are executed after 3-4 days. One cannot exclude that the addition to the growth medium of some ingredients conserving the cell phenotype could increase the number of passages applicable for the accumulation of the vaccine antigens.

In the case when it proved impossible to obtain from the tumor tissue the sufficient quantity of cells it would be necessary to perform their propagation by the cultivation until the necessary quantity is reached, the control of variability of the surface antigens during the cell cultivation remains obligatory. The control of the antigenic composition during the cultivation process is performed according to this invention without the additional cell propagation. For example, the non-deadly protease treatment and the mass spectrometry analysis are executed once or twice per week during the process of cultivation.

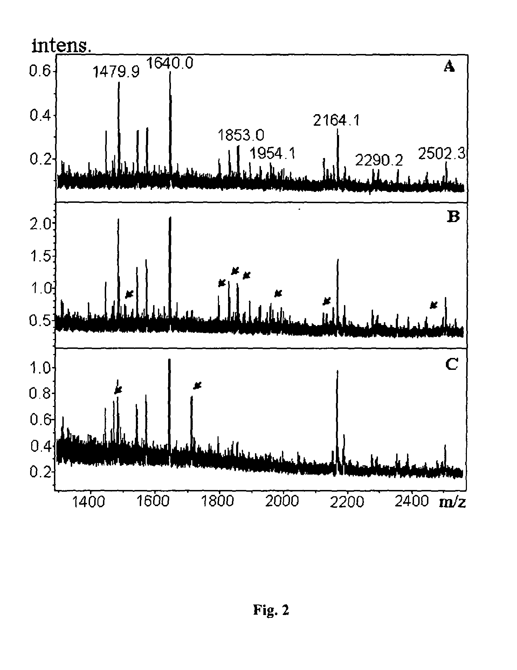

The identity of tumor and vaccine antigens secures the specificity of the anti-tumor immune response induced with the vaccine. The comparison of mass spectra of antigens of the original tumor and of antigens accumulated according to this invention permits to control such identity. The fig.2A represents the mass spectrum of surface antigens of original tumor cells. The fig.2B represents the mass spectrum of antigens suitable for the vaccination, which were obtained according this invention (mass spectra on the fig. 2A and 2B are identical). The fig.2C also shows the mass spectrum of antigens obtained according this invention, but not suitable for the vaccination (spectra on the fig.2A and 2C significantly differ). Arrows point to the masses corresponding to the disappeared (fig.2B) and newly appeared antigens (fig.2C). So controlling the peptide composition, it is possible to accumulate the quantity of antigens necessary for the vaccination, which induces the specific anti-tumor immune response.

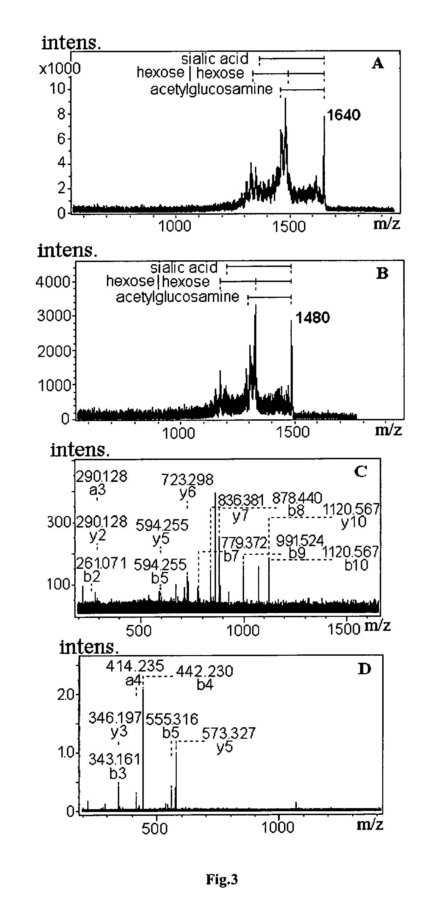

For the corroboration of the origination of the antigens from the surface proteins of tumor cells (though it is not necessary for the realization of invention) their fragmentation spectra were obtained, example of which are represented on the fig.3.

For this purpose the samples, obtained during the process of incubation of the cells with the trypsin, were desalted using tips of the automatic pipettes with the reverse phase ZipTip_{C18} (Millipore Corp., USA) in accordance with the protocol of manufacturer and they were applied on the mass spectrometer target with the matrix as exposed before. Mass spectra were registered in the regime of ion fragmentation. The identification of the proteins was performed using search system Mascot (MatrixScience, USA) upon the taxon *Homo sapiens* of the database of protein sequences NCBI (USA) with the use of ion masses 'b' and 'y' (*sequence-tag* method) and/or with the use of established amino acid sequences (*de novo* method).

Fig. 3A and 3B show examples of dissociation of the carbohydrate parts of glycosylated peptides with the masses 1640 and 1480 Da, the fragments with the masses 162, 203 and 291 Da were split, which corresponds to hexoses (mannose, glucose or galactose), acetylglucosamine and sialic acid, respectively. The fragmentation of peptides allowed identify their amino acid sequences and reveal in the antigens mixture the peptides from variable and constant domains of the heavy chain of immunoglobulins (which wear most of CD antigens, complexes of histocompatibility, T-cell receptor and molecules of the cell adhesion), the low-density lipoprotein receptor, the receptor of interleukin IR-2, G protein-bound receptor, the main histocompatibility complexes I and II. The fig.3C shows the example of the fragments of dissociation with the indication of b-y ions for the two-charge peptide with the mass 857.4 Da, with established amino acid sequence corresponding to the fragment of G protein-bound receptor:

Ile Cys Cys Ala Cys Cys Leu Cys Arg Asn Asn Cys Cys Val Phe Arg
1               5                   10                  15

The fig.3D shows another example of fragments of dissociation of one-charge peptide with the mass 573.3 Da with the indication of b-y ions and with established amino acid sequence which corresponds to the fragment of the main hystocompatibility complex II:

Ala Val Leu Asn Arg
1               5

So the antigens obtained accordingly to this invention are characterized with the following:
1) they are composed of water-soluble, mainly glycosylated, proteolitic peptides (their sequence usually ends with lysine or arginin when trypsin is used as protease) having masses from 1.2 to 4 kDa,
2) they are originating from one source - the extracellular fragments of the proteins of external plasmatic membrane of tumor cells,
3) they are obtained by means of treatment of the live cells with the protease in non-deadly concentration.

The practical use of the mixture of tumor antigens obtained according to this invention is stipulated by the identity of amino acid sequences and modifications (glycosylation) of the peptides of the mixture with protein fragments exhibited on the cell surface. It is well known that the emergence of oncological disease is a consequence of the inability of the immune system to destroy tumor cells arising in the organism. The accumulated and purified antigens mixed with the adjuvant which enhances the immune response is injected into the human or animal organism. As a result of cell and humoral immune responses of the organism which annihilates the injected peptides the already existing or newly appeared tumor cells are cross-destroyed, which stipulates the curing and prophylactic effects of anti-tumor vaccination. In order to obtain the full value immune response repeated injection of the antigens are used.

For the corroboration of the anti-tumor activity of the vaccine obtained according to this invention a model experiment was performed using 20 BALB/c mice males of the same age and weight (10 animals in the control and 10 animals in the test group).

The tumor antigens were obtained, according to the first variant of realization of the invention, from the hepatoma H22 cell culture. The collected antigens were desalted with the gel filtration through Sephadex G-10 and were concentrated on the vacuum concentrating device SpeedVac.

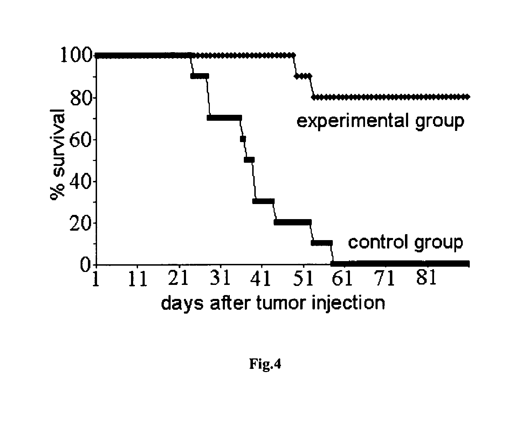

150 µg of preliminarily mixed with the complete Freund's adjuvant in ratio 1:1 (vol/vol) antigens were injected subcutaneously. The repeated immunizations were performed during three weeks (one injection per week) using the incomplete Freund's adjuvant. Animals of the control group were injected with Freund's adjuvant mixed with the physiological solution according to the same scheme. After the fourth immunization the hepatoma H22 was inoculated to mice by means of subcutaneous injection of 1 million cells. During three months the survival rate of animals in the control and test groups was checked. The obtained curve of survival (fig.4) corroborated the presence of anti-tumor activity of the antigens obtained according to the invention.

It is necessary to note that the results of the model experiment do not restrict the successful use of the invention by animals, because of the identity of the mechanisms of anti-tumor immunity in animals and in humans. The method of administration of the antigen and the antigen's dose calculated per kg weight keep their value in cases of the immunotherapy of humans, but the scheme of treatment could be individualized for each patient according to the severity of disease, the stage of disease, the mutability of tumor cells, the ability of the organism to give an obvious immune response to the antigen etc.

Further is given an example of the anti-tumor vaccine and the method of performing the anti-tumor therapy, on the base of surface tumor antigens, prepared according the second variant of realization of the invention, for the execution of anti-tumor therapy of humans.

The vaccine contains 1 mg of the mixture of tumor antigens, obtained according to the second variant of realization of invention, dissolved in 0.5 ml of phosphate buffer silane and mixed with 1 ml of the adjuvant Montanid ISA-51 (adjuvant of the firm Syntex on the base of oil-water emulsion containing squalene, Plunoric L121, Tween-80).

The one dose of vaccine is injected subcutaneously to the patient during three weeks - weekly and during five months - monthly.

The effectiveness of the vaccination is assessed by the intensity of immunity to introduced tumor antigens. 2-3 days after the injection the reaction of hypersensitivity to the injected antigens is assessed judging dimension of the red spot in the injection place. As a base value is accepted the dimension of the red spot which appeared after the first injection. The obvious intensification of the hypersensitivity following the repeated vaccinations indicates the developing anti-tumor immune response.

In other variants of realization of the method of performing the anti-tumor immunotherapy the intravenous or intramuscular injections are possible as also the injection of the vaccine without adjuvants.

The development of the immune response through the use of this invention is secured because of application of the mixture of tumor antigens. The plurality of peptides present in the vaccine warrants the possibility to receive the immune response to every cells which have on their surface proteins with the same amino acid sequences.

So the present invention allows increase many times the effectiveness of the anti-tumor immunotherapy in comparison with well-known methods where monovalent vaccines are used.

The employment of this group of inventions secures the possibility of performing the anti-tumor immunotherapy using the vaccine enriched with tumor-specific antigens that is surface antigens of the tumor cells.

This possibility is created because of treatment of tumor cells with a protease applied in non-deadly for the cells concentration. The use of the non-deadly concentrations of the protease permits to separate only the surface antigens of the tumor cells and allows to avoid the cell death which is accompanied by the destruction of their cytoplasmic membranes, which leads to the emergence of the cytoplasm contents in the vaccine, in particular to the emergence of a mass of intracellular proteins having no use for the vaccination. This situation leads to the decrease of the part of tumor specific antigens in the well-known vaccines, to the "blurring" of the immune response and as a consequence to the decrease of the immune response specificity namely to the tumor antigens.

So the use of the vaccine enriched with tumor antigens, prepared according to the present invention, allows to rise the effectiveness of the anti-tumor immunotherapy.

Moreover the rise of effectiveness of the immunotherapy is reached because of the use of antigens obtained by the accumulation out of the primary tumor cell culture. This method of preparation of the anti-tumor vaccine permits to obtain after every protease treatment the antigens which practically possess the unchanged composition and to accumulate the quantity of antigens necessary for the vaccination using the primary cell culture. The employment in the vaccine composition of tumor antigens specific only for the given tumor is secured because of the possibility to control the composition of the accumulated antigens.

Also is excluded the possibility of appearance of antigens non-specific for the given tumor in the vaccine. Such antigens can be present in the composition of well-known vaccines prepared with the use of proliferated cells, because it is known that primary cultures during the proliferation *in vitro* change their antigenic composition. So the composition of the mixture of antigens collected from the proliferated cells could significantly differ from the composition of antigens obtained from the isolated (not cultivated) tumor cells.

The vaccines prepared according to the present invention could be autologous vaccines (obtained from the patient's own cells) or allogeneic vaccines (on the base of the cells of another patient).

The immunotherapy performed accordingly to this invention is effective in both cases, because tumor cells of different patients have on their surfaces peptides with identical amino acid sequences.

Also the use of auto-vaccines prepared accordingly to this invention is more effective because in the latter case the vaccine contains individual and stage-specific antigens distinctive for the development of the tumor process of the concrete patient.

### INDUSTRIAL APPLICABILITY

Thus the invention allows to obtain the tumor antigens on the base of surface cell antigens and allows to perform on their base the treatment of oncological diseases, namely the vaccination.

## Claims

1. An antitumor vaccine based on surface tumor antigens, **characterized in that**
the vaccine comprises a mixture of surface tumor antigens, said antigens being accumulated peptides from surface proteins of life tumor cells, obtained by a periodic and a non-deadly protease treatment of the cells of a primary culture of the life tumor cells.

2. An antitumor vaccine according to claim 1, **characterized in that** the vaccine is obtained under trypsin treatment, wherein trypsin is selected for protease.

3. A method of preparation of the anti-tumor vaccine including the cultivation of the tumor cells and the isolation of surface tumor antigens **characterized in that** the primary tumor cell culture, previously washed from the growth medium, is subjected to the non-deadly treatment with the protease, the released surface tumor antigens are collected and the treatment of the primary tumor cell culture by protease is repeated after time intervals necessary for the recovery of surface antigens by the tumor cells, the surface tumor antigens are accumulated until their dose necessary for the vaccination is reached, the composition of the accumulated surface tumor antigens is controlled.

4. A method according to claim 3, **characterized in that** trypsin is used as protease.

5. A method of conducting an anti-tumor immunotherapy, comprising a step of introduction into an organism of the patient a vaccine obtained from a mixture of a surface tumor antigens, being peptides, obtained from surface proteins of tumor cells, **characterized in that** the mixture of the surface tumor antigens is used, said antigens being accumulated peptides from surface proteins of life tumor cells, obtained by a periodic and a non-deadly for the cells protease treatment of a primary culture of life tumor cells.

6. A method according to claim 5, wherein trypsin is used for protease.

7. A method according to claim 5, wherein surface antigens of autologous tumor cells are used.

8. A method according to claim 5, wherein surface antigens of allogeneic tumor cells are used.

9. A method according to claim 5, wherein the antitumor vaccine comprises adjuvants.
